# EUROPEAN PATENT SPECIFICATION

(11) **EP 3 638 861 B1**
(45) Date of publication and mention of the grant of the patent: **06.05.2026**
(21) Application number: 18817153.2
(22) Date of filing: 12.06.2018
(51) Int. Cl.: E03C 1/126, A47K 3/28, A61L 2/16, A61L 2/18, E03B 1/04, E03C 1/02, C02F 1/68, C02F 1/50, C02F 1/00, E03C 1/04

(54) **A METHOD FOR DISINFECTION AND CLEANING OF AT LEAST ONE PART OF A DEVICE INTENDED FOR RECYCLING OF WATER**
VERFAHREN ZUR DESINFEKTION UND REINIGUNG VON WENIGSTENS EINEM TEIL EINER VORRICHTUNG ZUM RECYCLING VON WASSER
PROCÉDÉ DE DÉSINFECTION ET DE NETTOYAGE D'AU MOINS UNE PARTIE D'UN DISPOSITIF DESTINÉ AU RECYCLAGE DE L'EAU

(30) Priority: 16.06.2017 SE 1750768
(43) Date of publication of application: 22.04.2020
(73) Proprietor: Orbital Systems AB, 211 20 Malmö (SE)
(72) Inventor: REHN, Gustav, 224 72 Lund (SE); ESKILANDER, Stephan, 224 75 Lund (SE)
(74) Representative: AWA Sweden AB
(86) International application number: PCT/SE2018/050610
(87) International publication number: WO 2018/231131

(56) References cited:
- WO-A1-01/94513
- WO-A1-2009/147647
- WO-A1-2015/071244
- DE-A1- 10 033 479
- JP-A- 2004 136 266
- RU-C1- 2 275 835
- US-A1- 2003 083 219
- US-A1- 2003 096 725
- US-A1- 2009 165 228
- US-A1- 2010 237 021
- US-A1- 2011 146 800
- US-A1- 2013 079 408
- US-A1- 2013 212 800
- US-A1- 2013 212 800
- US-A1- 2013 239 991
- US-A1- 2015 344 323
- US-A1- 2017 035 049

## Description

### Field of the invention

The present invention relates to a method for disinfection and cleaning of a water recycling device.

### Technical Background

There are different water recycling devices known today. One example is shown in WO 2013/095278 where there is disclosed a hybrid device for a recirculation shower, allowing purification and either recycling of water or discarding of water. The hybrid device comprises a recirculation loop, a filter system with a pre-filter and a nano-filter, at least one filter quality sensor, and the device is arranged to redirect the water from recirculation to drainage when the at least one filter quality sensor indicates the need thereof.

Another example is US 2010/237021 A1 describing a method of cleaning and disinfecting a recirculating shower.

The present invention is directed to a disinfection and cleaning method suitable for water recycling devices. The water recycling devices are recirculating showers, recirculating dishwashers or recirculating washing machines. The method of the present invention is directed to the device being a recirculating shower.

The method is especially suitable for water recirculating devices used domestically, but also other usage is totally possible. Examples are recirculating showers systems used in hotels and bathhouses. Fact is that any type of such system, used industrially or domestically, is of interest for the method according to the present invention.

Moreover, it should be noted that the common feature of the water recirculating systems intended above is the possibility of recirculating water to be recycled and reused. Water being contaminated, or a liquid fraction containing contaminants, however, should be flowed to a drain. Also this fraction may be recycled in another system or collected depending on the kind of system and type of contaminants.

The present invention is directed to providing an optimal disinfection and cleaning method for water recirculating showers.

### Summary of the invention

The latter stated purpose above is achieved by a method for disinfection and cleaning of at least one part of a device intended for recycling of water, said method involving flowing lactic acid, citric acid and/or an enzyme or surfactant based solution into the part intended to receive the disinfection and cleaning. The method of the present invention is defined in the appended claims.

In relation to the method according to the present invention it should be mentioned that the method involves both disinfection implying killing or inactivating bacteria and microorganisms, and also cleaning implying to remove dead or inactivated bacteria and microorganisms from tubing etc. The latter step can be seen as an antifouling step, so that the material is possible to be flush out from the water recirculating system or device so such material is not accumulated in the system.

### Specific embodiments of the invention

Below there are disclosed and discussed different embodiments of the method according to the present invention.

According to one specific embodiment of the present invention, the method involves at least flowing lactic acid into the part intended to receive disinfection treatment. Lactic acid may be used alone or in combination with other substances. According to one specific embodiment, the method involves flowing both an antibacterial agent for the disinfection and also an antifouling agent for the cleaning into the part intended to receive the disinfection and cleaning. Also in this case the antibacterial agent may be lactic acid. Furthermore, the flowing of the antibacterial agent and antifouling agent may be performed simultaneously or sequentially.

The substances intended to be used in the method according to the present invention have the advantage of being environmental friendly and biologically degradable to a large extent. As the disinfection and cleaning method according to the present invention is intended to be used in devices intended to save water and energy the aspect above is of great importance. The usage of environmental friendly and biologically degradable substances which has a very low negative impact on water and energy loss and use of chemicals is of course preferable in water recirculating systems, and in fact also in other systems where water is used and some amounts are wasted.

Moreover, both lactic acid and citric acid, and also several enzyme or surfactant based substances are easy to use and inexpensive.

The device intended for method comprises a flow path for recycled water. Other units of the device are a fresh water inlet, a recirculation water inlet and a recirculation water outlet, a user outlet, a pump, sensors, valves, a heater and a filter. According to the present invention the method involves flowing lactic acid, citric acid and/or an enzyme based or surfactant based solution into at least part of the flow path for recycled water. One part of special interest for the method is the user outlet. In this part and the tubing in close proximity to the user outlet there is an evident risk for growth of bacteria and microorganisms. Therefore, this part is of special interest for the method according to the present invention. In line with this, the process order subsequent to the filter is the heater and then finally the user outlet and wherein flowing of lactic acid, citric acid and/or an enzyme based or surfactant based solution is performed into at least the user outlet of the device. Also in this case only lactic acid may be used, but also in combination with an enzyme based and/or surfactant based solution.

The flowing of lactic acid, citric acid and/or an enzyme or surfactant based solution is provided from the filter into the system and thus into all of the system arranged subsequent to the filter, also comprising the heater and the user outlet.

The provision of the substance(s) into the system is provided in a manner wherein a disposable filter unit is replaced with a cartridge containing the lactic acid, citric acid and/or an enzyme or surfactant based solution for allowing for performing the disinfection treatment, or wherein the filter is a disposable filter unit also comprising a dosing capsule containing lactic acid, citric acid and/or an enzyme based or surfactant based solution and wherein the dosing capsule discharges the content when the filter is pressurized. The cartridge is of course also a disposable to be used for the disinfection and cleaning of the system. When the disposable filter unit is replaced with a cartridge, the time point for performing the disinfection and cleaning may vary, but is intended to be performed at a decided time. This can be set by the system as such, e.g. when sensors are giving data to the processor that it is time for performing a disinfection and cleaning cycle. In such a case the system will inform a user that the method should be performed. Also a set certain use period (weeks, months) or a number of use cycles may be the parameters deciding when the method for disinfecting and cleaning should be performed.

The dosing of the lactic acid, citric acid and/or an enzyme or surfactant based solution may be performed during a time sequence in the beginning of usage of a new filter or may in fact be dosed based on a slow release technique so that the substance(s) are added continuously into the system at a suitable amount. According to the invention, the device intended for usage and recycling of water is a recirculating shower. As should be evident, when the disinfection and cleaning method is being performed, then regular use is not performed at the same time.

### Detailed description of the drawings

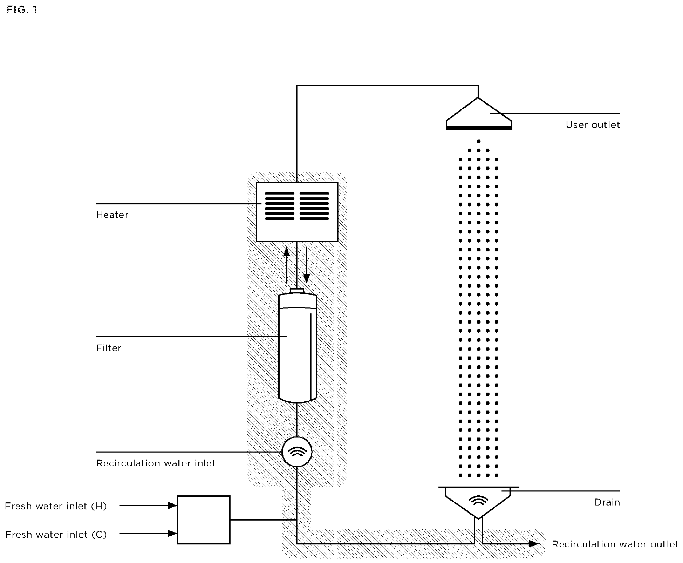

In fig. 1 there is shown a water recirculating device, in this case a recirculating shower, in which the method according to one embodiment of the present invention may be used. As seen in fig. 1, the device comprises a fresh water inlet, both cold and hot, the water flowed in here meets a recirculation loop comprising a recirculation water inlet, which in fact is one point along the loop before subsequent key units being a filter and then a heater. In the flow path up-streams, the user outlet is provided subsequently to the heater. As seen in fig. 1 there is arranged a drain being a point from which water is either wasted via the water recirculation outlet or sent to recirculation. As said above, to perform the method according to the present invention, the opening of valves (not shown) may be used. Moreover, sensors providing data to the processing unit and also a pump providing recirculating force are other units not shown in fig. 1.

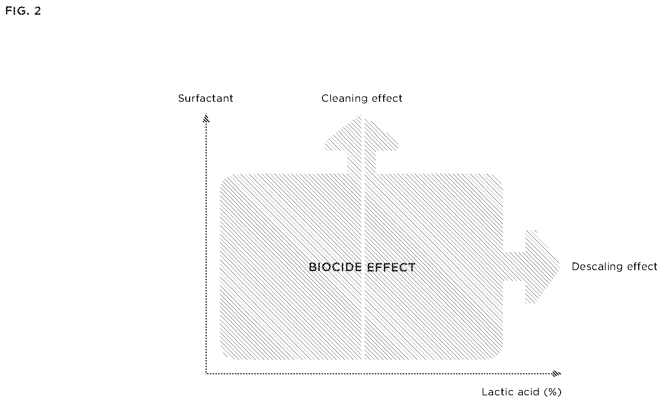

In fig. 2 there is shown a schematic view of the disinfecting (descaling) effect and cleaning effect when using lactic acid as the disinfecting agent and a surfactant as the cleaning agent according to the present invention.

## Claims

1. A method for disinfection and cleaning of at least one part of a device intended for recycling of water, said method involving flowing lactic acid, citric acid and/or an enzyme based or surfactant based solution into the part intended to receive the disinfection and cleaning;
wherein the device comprises a flow path for recycled water, a fresh water inlet, a recirculation water inlet and a recirculation water outlet, a user outlet, a pump, sensors, valves, a heater and a filter and wherein the method involves flowing lactic acid, citric acid and/or an enzyme based or surfactant based solution into at least part of the flow path for recycled water,
wherein the user outlet is one part being disinfected and cleaned,
wherein the device intended for usage and recycling of water is a recirculating shower,
and wherein the process order subsequent to the filter is the heater and then finally the user outlet and wherein flowing of lactic acid, citric acid and/or an enzyme based or surfactant based solution is performed into at least the user outlet of the device,
wherein the flowing of lactic acid, citric acid and/or an enzyme based or surfactant based solution is provided from the filter into the system and thus into all of the system arranged subsequent to the filter, also comprising the heater and the user outlet, in a manner;
wherein a disposable filter unit is replaced with a cartridge containing the lactic acid, citric acid and/or an enzyme based or surfactant based solution for allowing for performing the disinfection treatment, or
wherein the filter is a disposable filter unit also comprising a dosing capsule containing lactic acid, citric acid and/or an enzyme based or surfactant based solution and wherein the dosing capsule discharges the content when the filter is pressurized.

2. The method according to claim 1, wherein the method involves at least flowing lactic acid into the part intended to receive disinfection treatment.

3. The method according to claim 1 or 2, wherein the method involves flowing both an antibacterial agent for the disinfection and also an antifouling agent for the cleaning into the part intended to receive the disinfection and cleaning.

## Patentansprüche

1. Verfahren zur Desinfektion und Reinigung von mindestens einem Teil einer Vorrichtung, die für das Recycling von Wasser vorgesehen ist, wobei das Verfahren das Zuführen von Milchsäure, Zitronensäure und/oder einer Lösung auf Enzymbasis oder Tensidbasis in das zur Desinfektion und Reinigung bestimmte Teil einschließt;
wobei die Vorrichtung einen Strömungsweg für recyceltes Wasser, einen Frischwassereinlass, einen Umwälzwassereinlass und einen Umwälzwasserauslass, einen Benutzerauslass, eine Pumpe, Sensoren, Ventile, ein Heizelement und einen Filter umfasst und wobei das Verfahren das Zuführen von Milchsäure, Zitronensäure und/oder einer Lösung auf Enzymbasis oder Tensidbasis in zumindest einen Teil des Strömungsweges für recyceltes Wasser einschließt,
wobei der Benutzerauslass ein Teil ist, das desinfiziert und gereinigt wird,
wobei das Gerät für die Verwendung und das Recycling von Wasser eine Umwälzdusche ist,
und wobei die Prozessordnung nach dem Filter das Heizelement und dann schließlich der Benutzerauslass ist und wobei das Zuführen von Milchsäure, Zitronensäure und/oder einer enzymatischen oder Tensidlösung in zumindest den Benutzerauslass der Vorrichtung erfolgt,
wobei das Zuführen von Milchsäure, Zitronensäure und/oder einer Lösung auf Enzymbasis oder Tensidbasis vom Filter in das System und damit in das gesamte nach dem Filter angeordnete System, welches auch das Heizelement und den Benutzerauslass umfasst, auf eine Weise erfolgt;
wobei eine Einwegfiltereinheit durch eine Kartusche ersetzt wird, die die Milchsäure, Zitronensäure und/oder eine Lösung auf Enzymbasis oder Tensidbasis enthält, um die Durchführung der Desinfektionsbehandlung zu ermöglichen, oder
wobei der Filter eine Einwegfiltereinheit ist, die zudem eine Dosierkapsel umfasst, welche Milchsäure, Zitronensäure und/oder eine Lösung auf Enzymbasis oder Tensidbasis enthält, und wobei die Dosierkapsel den Inhalt abgibt, wenn der Filter mit Druck beaufschlagt wird.

2. Verfahren nach Anspruch 1, wobei das Verfahren mindestens das Zuführen von Milchsäure in das zur Desinfektionsbehandlung bestimmte Teil einschließt.

3. Verfahren nach Anspruch 1 oder 2, **dadurch gekennzeichnet, dass** das Verfahren sowohl das Zuführen eines antibakteriellen Mittels zur Desinfektion als auch eines Antifouling-Mittels zur Reinigung in das zur Desinfektion und Reinigung vorgesehene Teil einschließt.

## Revendications

1. Procédé de désinfection et de nettoyage d'au moins une partie d'un dispositif destiné au recyclage de l'eau, ledit procédé impliquant de faire s'écouler de l'acide lactique, de l'acide citrique et/ou une solution à base d'enzyme ou de tensioactif dans la partie destinée à recevoir la désinfection et le nettoyage ;
le dispositif comprenant une trajet d'écoulement pour l'eau recyclée, une entrée d'eau propre, une entrée d'eau de recirculation et une sortie d'eau de recirculation, une sortie d'utilisateur, une pompe, des capteurs, des vannes, un réchauffeur et un filtre, et le procédé impliquant de faire s'écouler de l'acide lactique, de l'acide citrique et/ou une solution à base d'enzyme ou de tensioactif dans au moins une partie du trajet d'écoulement pour l'eau recyclée,
la sortie utilisateur étant une partie en cours de désinfection et de nettoyage,
le dispositif destiné à l'utilisation et au recyclage de l'eau étant une douche à recirculation,
et l'ordre de traitement suivant le filtre étant le réchauffeur et finalement la sortie utilisateur et l'écoulement de l'acide lactique, de l'acide citrique et/ou d'une solution à base d'enzymes ou de tensioactif étant effectué dans au moins la sortie utilisateur du dispositif,
l'écoulement de l'acide lactique, de l'acide citrique et/ou d'une solution à base d'enzymes ou de tensioactif étant assuré à partir du filtre dans le système et donc dans tout le système disposé après le filtre, comprenant également le réchauffeur et la sortie d'utilisateur, d'une manière ;
une unité filtrante jetable étant remplacée par une cartouche contenant l'acide lactique, l'acide citrique et/ou une solution à base d'enzyme ou de tensioactif pour permettre la réalisation du traitement de désinfection, ou
le filtre étant une unité filtrante jetable comprenant également une capsule doseuse contenant de l'acide lactique, de l'acide citrique et/ou une solution à base d'enzyme ou de tensioactif, et la capsule doseuse déchargeant le contenu lorsque le filtre est sous pression.

2. Procédé selon la revendication 1, le procédé impliquant au moins l'écoulement d'acide lactique dans la partie destinée à recevoir un traitement de désinfection.

3. Procédé selon la revendication 1 ou 2, le procédé impliquant l'écoulement à la fois d'un agent antibactérien pour la désinfection et également d'un agent antisalissure pour le nettoyage dans la partie destinée à recevoir la désinfection et le nettoyage.
